# EUROPEAN PATENT SPECIFICATION

(11) **EP 3 599 906 B1**
(45) Date of publication and mention of the grant of the patent: **19.05.2021**
(21) Application number: 18713913.4
(22) Date of filing: 27.03.2018
(51) Int. Cl.: A24F 47/00, A61M 15/06

(54) **CARTRIDGE ASSEMBLY COMPRISING A BLOCKING ELEMENT**
PATRONENANORDNUNG MIT EINEM SPERRELEMENT
ENSEMBLE DE CARTOUCHE COMPRENANT UN ÉLÉMENT DE BLOCAGE

(30) Priority: 29.03.2017 EP 17163666
(43) Date of publication of application: 05.02.2020
(73) Proprietor: Philip Morris Products S.A., 2000 Neuchâtel (CH)
(72) Inventor: PATORET, Véronique, 69170 Joux (FR)
(74) Representative: Dowling, Ian
(86) International application number: PCT/EP2018/057806
(87) International publication number: WO 2018/178092

(56) References cited:
- WO-A1-2008/121610
- WO-A1-2015/197627
- WO-A2-2013/184951

## Description

The present invention relates to a cartridge assembly for use in an aerosol-generating system and an aerosol-generating system comprising the cartridge assembly. The invention finds particular application as a cartridge assembly comprising a nicotine source and an acid source for the generation of an aerosol comprising nicotine salt particles.

Devices for delivering nicotine to a user and comprising a nicotine source and a volatile delivery enhancing compound source are known. For example, WO 2008/121610 A1 discloses devices in which nicotine and a volatile acid, such as pyruvic acid, are reacted with one another in the gas phase to form an aerosol of nicotine salt particles that is inhaled by the user.

In WO 2008/121610 A1 the nicotine source and volatile delivery enhancing compound source may be housed in compartments that are sealed by removable or frangible barriers prior to initial use of the aerosol-generating system.

WO 2015/197627 A1 describes an aerosol-generating system comprising a cartridge having a first compartment containing a nicotine source and a second compartment containing a lactic acid source, wherein the first and second compartments are sealed by frangible barriers.

However, the inclusion of multiple removable or frangible barriers may disadvantageously increase the cost and complexity of manufacturing such aerosol-generating systems. Consequently, it would be desirable to provide a cartridge assembly for use in an aerosol-generating system in which one or more volatile compounds may be retained during storage and which minimises the need for removable or frangible barriers.

According to a first aspect of the present invention there is provided a cartridge assembly for use in an aerosol-generating system, the cartridge assembly comprising a mouthpiece housing, a blocking element and a cartridge. The mouthpiece housing has an upstream end and a downstream end, the mouthpiece housing defining a mouthpiece cavity extending between the upstream end of the mouthpiece housing and the downstream end of the mouthpiece housing. The blocking element is secured within the mouthpiece cavity and comprises an upstream end and a downstream end. The cartridge is positioned at the upstream end of the mouthpiece housing, the cartridge comprising an upstream end and a downstream end. The cartridge also comprises a first compartment having a first air inlet at the upstream end of the cartridge and a first air outlet at the downstream end of the cartridge. The cartridge also comprises a second compartment having a second air inlet at the upstream end of the cartridge and a second air outlet at the downstream end of the cartridge. The downstream end of the cartridge abuts the upstream end of the blocking element. The cartridge is rotatable with respect to the blocking element from a first position to a second position. The blocking element is shaped so that the upstream end of the blocking element obstructs the first air outlet and the second air outlet when the cartridge is in the first position, and the first air outlet and the second air outlet are both in fluid communication with the mouthpiece cavity when the cartridge is in the second position.

As used herein with reference to the invention, the term "air inlet" is used to describe one or more apertures through which air may be drawn into a component or portion of a component of the cartridge assembly.

As used herein with reference to the invention, the term "air outlet" is used to describe one or more apertures through which air may be drawn out of a component or portion of a component of the cartridge assembly.

As used herein with reference to the invention, by "obstructed" it is meant that an air inlet or an air outlet is blocked such that airflow through the air inlet or the air outlet is substantially prevented.

The cartridge assembly according to the present invention comprises a blocking element configured to obstruct the first and second air outlets of the cartridge when the cartridge is in the first position. Advantageously, this can eliminate the need to provide a removable or frangible seal at the downstream end of the cartridge.

The cartridge assembly according to the present invention comprises a cartridge configured to rotate with respect to the blocking element from a first position in which the blocking element obstructs the first and second air outlets to a second position in which the first and second air outlets are in fluid communication with the mouthpiece cavity. Advantageously, this can simplify use of the cartridge assembly by allowing a user to uncover the first and second air outlets without the need to remove a seal or break a seal at the downstream end of the cartridge.

The cartridge assembly may be configured so that the cartridge is rotatable with respect to the blocking element between the first position and the second position. That is, the cartridge may be configured for rotation from the first position to the second position and configured for rotation from the second position to the first position. This may allow a user to rotate the cartridge from the second position to the first position to re-obstruct the first and second air outlets. Advantageously, this may reduce the loss of one or more volatile compounds that may be stored within at least one of the first compartment and the second compartment between separate uses of the cartridge.

One of the blocking element and the cartridge may comprise an aperture, wherein the other of the blocking element and the cartridge comprises a shaft, and wherein at least a portion of the shaft is positioned within the aperture. Advantageously, the combination of the shaft and the aperture facilitates rotation of the cartridge with respect to the blocking element. Advantageously, the combination of the shaft and the aperture may be simple to manufacture and assemble.

Preferably, the aperture forms a plain bearing.

The aperture may extend through the blocking element between the upstream end of the blocking element and the downstream end of the blocking element, wherein the shaft extends from the downstream end of the cartridge. Advantageously, this arrangement may facilitate embodiments in which a length of the shaft is longer than a length of the aperture. In particular, the mouthpiece cavity may accommodate any portion of the shaft which extends beyond the downstream end of the blocking element.

Preferably, the shaft comprises a journal portion positioned within the aperture and a retaining portion at an end of the journal portion, wherein the retaining portion is positioned outside of the aperture and has a diameter that is larger than a diameter of the aperture. Advantageously, the retaining portion retains the journal portion of the shaft within the aperture and prevents separation of the cartridge from the blocking element and the mouthpiece housing.

The blocking element may comprise a first part defining a first side of the aperture and a second part defining a second side of the aperture, wherein the first and second parts are formed separately and joined together to form the blocking element. Advantageously, forming the blocking element from two separate parts may facilitate assembly of the cartridge assembly. For example, the journal portion of the shaft may be positioned within at least one of the first side of the aperture and second side of the aperture before the first and second parts are joined together to form the blocking element.

The first and second parts may be joined together by at least one of an interference fit, an adhesive, and a weld, such as an ultrasonic weld.

The retaining portion may comprise a tapered portion. Advantageously, providing the retaining portion with a tapered portion may facilitate insertion of the retaining portion through the aperture during assembly of the cartridge assembly, particularly in embodiments in which the blocking element is formed as a unitary element. In such embodiments, preferably the maximum diameter of the tapered portion is sufficiently small so that elastic deformation of the material forming the blocking element can accommodate the retaining portion when it is pushed through the aperture during assembly of the cartridge assembly.

Preferably, the cross-sectional size of the tapered portion increases in the upstream direction along the length of the tapered portion from a downstream end of the tapered portion. Advantageously, this may facilitate insertion of the retaining portion into the aperture during assembly of the cartridge assembly. Preferably, an upstream end of the tapered portion has a diameter that is larger than a diameter of the aperture. Preferably, the downstream end of the tapered portion has a diameter that is smaller than a diameter of the aperture. The tapered portion may comprise a cross-sectional size that varies linearly along the length of the tapered portion. For example, the tapered portion may have a conical shape. The tapered portion may comprise a cross-sectional size that varies non-linearly along the length of the tapered portion. For example, the tapered portion may have a rounded shaped.

Preferably, the shaft comprises a step change in diameter between the upstream end of the tapered portion and the downstream end of the journal portion, wherein the downstream end of the journal portion has a diameter that is smaller than the diameter of the upstream end of the tapered portion. Advantageously, a step change in diameter may prevent the tapered portion being pulled back through the aperture after the cartridge assembly has been assembled.

The cartridge may comprise a guiding pin extending from the downstream end of the cartridge, wherein the guiding pin abuts a first portion of the blocking element when the cartridge is in the first position, and wherein the guiding pin abuts a second portion of the blocking element when the cartridge is in the second position. Advantageously, the guiding pin may facilitate precise rotation of the cartridge into the second position by a user. In embodiments in which the cartridge can also be rotated from the second position into the first position, the guiding pin may facilitate precise rotation of the cartridge into the first position by a user. Preferably, the guiding pin is spaced apart from an axis of rotation of the cartridge with respect to the blocking element so that the guiding pin follows an arcuate path when the cartridge is rotated from the first position to the second position.

Preferably, the blocking element comprises a guide opening extending through the blocking element between the upstream end of the blocking element and the downstream end of the blocking element, wherein the guiding pin is received within the guide opening. Preferably, the guiding pin abuts a first side of the guide opening when the cartridge is in the first position. Preferably, the guiding pin abuts a second side of the guide opening when the cartridge is in the second position.

The blocking element may comprise a first recess formed in the first side of the guide opening, wherein the guiding pin is received within first recess by an interference fit when the cartridge is in the first position. Advantageously, the interference fit may cause the guiding pin to snap into the first recess when the cartridge is moved into the first position from the second position. This may provide a user with a tactile confirmation that the cartridge has been successfully rotated into the first position. The blocking element may comprise a first detent extending into the guide opening to provide the interference fit between the first recess and the guiding pin.

The blocking element may comprise a second recess formed in the second side of the guide opening, wherein the guiding pin is received within second recess by an interference fit when the cartridge is in the second position. Advantageously, the interference fit may cause the guiding pin to snap into the second recess when the cartridge is moved into the second position from the first position. This may provide a user with a tactile confirmation that the cartridge has been successfully rotated into the second position. The blocking element may comprise a second detent extending into the guide opening to provide the interference fit between the second recess and the guiding pin.

The blocking element may comprise only the first recess, only the second recess, or both the first recess and the second recess. In embodiments in which the cartridge assembly is configured so that the cartridge may be rotated only from the first position to the second position, preferably the interference fit between the guiding pin and the second recess is substantially irreversible. That is, the force required to remove the guiding pin from the second recess may be at least double the force required to insert the guiding pin into the second recess. The interference fit may be such that the guiding pin cannot be rotated out of the second recess without breaking the guiding pin.

The cartridge may comprise a cartridge housing positioned at the upstream end of the mouthpiece housing and rotatable with respect to the blocking element from the first position to the second position, and a cartridge body secured within the cartridge housing, the cartridge body defining the first compartment and the second compartment. Advantageously, forming the cartridge from a cartridge housing and a cartridge body may simplify the manufacture of the cartridge.

Preferably, the cartridge housing comprises a downstream end wall.

Preferably, the first and second air outlets extend through the downstream end wall.

In embodiments in which the cartridge comprises a shaft, preferably the shaft extends downstream from the downstream end wall. Preferably, the shaft is formed integrally with the downstream end wall.

In embodiments in which the cartridge comprises a guiding pin, preferably the guiding pin extends downstream from the downstream end wall. Preferably, the guiding pin is formed integrally with the downstream end wall.

Preferably, the cartridge housing defines a cartridge housing cavity in which the cartridge body is received. Preferably, the cartridge housing defines a cartridge housing opening at an upstream end of the cartridge housing. Advantageously, the cartridge body may be inserted into the cartridge housing cavity through the cartridge housing opening.

The cartridge body and the cartridge housing may be shaped to limit the number of rotational orientations of the cartridge body with respect to the cartridge housing in which the cartridge body may be inserted into the cartridge housing. Preferably, the cartridge body and the cartridge housing are shaped so that the cartridge body may be inserted into the cartridge housing in only a single rotational orientation. In embodiments in which the first and second air outlets extend through a downstream wall of the cartridge housing, preferably the rotational orientation is such that, when the cartridge body is received within the cartridge housing, a downstream end of the first compartment overlies the first air aperture and a downstream end of the second compartment overlies the second air aperture.

The cartridge body may be retained within the cartridge housing by at least one of an interference fit, an adhesive, and a weld, such as an ultrasonic weld. Preferably, the cartridge body is retained within the cartridge housing by an interference fit. Advantageously, an interference fit simplifies the manufacture and assembly of the cartridge.

The cartridge body may be formed as a single part.

The cartridge body comprise a first part and a second part joined to the first part to form the cartridge body. Advantageously, forming the cartridge body from first and second parts may facilitate the insertion of a material into at least one of the first compartment and the second compartment during assembly of the cartridge assembly.

The first part of the cartridge body may define a first side of the first compartment and a first side of the second compartment. The second part of the cartridge body may define a second side of the first compartment and a second side of the second compartment. Any materials to be positioned within the first compartment may be positioned within the first side of the first compartment or the second side of the first compartment before the first and second parts are joined together to form the cartridge body. Any materials to be positioned within the second compartment may be positioned within the first side of the second compartment or the second side of the second compartment before the first and second parts are joined together to form the cartridge body.

The first and second parts of the cartridge body may be joined together by at least one of an interference fit, an adhesive, and a weld, such as an ultrasonic weld. Preferably, the first and second parts of the cartridge body are joined together by an interference fit. Advantageously, an interference fit simplifies the manufacture and assembly of the cartridge body.

Preferably, the cartridge body comprises an upstream end wall, wherein the first and second air inlets are defined by the upstream end wall.

The blocking element may be spaced apart from the downstream end of the mouthpiece housing, wherein a portion of the mouthpiece cavity between the downstream end of the blocking element and the downstream end of the mouthpiece housing forms a mixing chamber. In embodiments described herein in which the first compartment comprises a source of a first volatile compound and the second compartment comprises a source of a second volatile compound, advantageously the mixing chamber may facilitate mixing of vaporised first and second volatile compounds from the first and second compartments before the mixture is delivered to a user.

The mouthpiece housing may comprises a ventilation air inlet positioned between the blocking element and the downstream end of the mouthpiece housing, the ventilation air inlet extending through the mouthpiece housing and providing fluid communication between the exterior of the mouthpiece housing and the mixing chamber.

The mouthpiece housing may comprise a downstream wall portion extending across the downstream end of the mouthpiece cavity, the mouthpiece housing comprising a mouthpiece housing air outlet extending through the downstream wall portion. In use, airflow through the cartridge assembly exits the cartridge assembly through the mouthpiece housing air outlet for delivery to a user.

The blocking element may be secured within the mouthpiece housing by at least one of an interference fit, an adhesive, and a weld, such as an ultrasonic weld. Preferably, the blocking element is secured within the mouthpiece housing by an interference fit. Advantageously, an interference fit simplifies the manufacture and assembly of the cartridge assembly.

The blocking element may comprise a first airflow aperture configured to overlie the first air outlet when the cartridge is in the second position and a second airflow aperture configured to overlie the second air outlet when the cartridge is in the second position.

Preferably, the blocking element has a circular shape. The first airflow aperture and the second airflow aperture may each have an arcuate shape.

In embodiments in which the blocking element comprises a guide opening configured to interact with a guiding pin on the cartridge, the first airflow aperture or the second airflow aperture may form the guide opening.

The first airflow aperture and the second airflow aperture may be diametrically opposed. Advantageously, providing diametrically opposed first and second airflow apertures may facilitate positioning of the first and second compartments on opposite sides of the cartridge. Advantageously, this may facilitate a balanced distribution of weight within the cartridge, which may facilitate handling of the cartridge and the cartridge assembly during manufacturing processes.

The first airflow aperture and the second airflow aperture may each extend along a quarter-circular arc length. In embodiments in which the first airflow aperture and the second airflow aperture are diametrically opposed and each extend along a quarter-circular arc length, the first position and the second position may be offset from each other by an angular rotation of the cartridge through 90 degrees with respect to the blocking element.

In embodiments in which the downstream end of the blocking element has a circular shape, the downstream end of the blocking element defines a circumferential direction and a radial direction. Preferably, each of the first and second airflow apertures has a maximum length in the circumferential direction of at least about 3.5 millimetres, more preferably at least about 4 millimetres. Preferably, each of the first and second airflow apertures has a maximum length in the circumferential direction of less than about 5.5 millimetres, more preferably less than about 5 millimetres.

Preferably, each of the first and second airflow apertures has a maximum width in the radial direction of at least about 2 millimetres, more preferably at least about 2.5 millimetres. Preferably, each of the first and second airflow apertures has a maximum width in the radial direction of less than about 4 millimetres, more preferably less than about 3.5 millimetres.

The blocking element may have a diameter of at least about 6 millimetres, preferably at least about 6.5 millimetres. The blocking element may have a diameter of less than about 9 millimetres, preferably less than about 8.5 millimetres.

Preferably, each of the first and second airflow apertures has a maximum flow area of at least about 3.5 square millimetres, more preferably at least about 4 square millimetres, more preferably at least about 4.5 square millimetres. Preferably, each of the first and second airflow apertures has a maximum flow area of less than about 6 square millimetres, more preferably less than about 5.5 square millimetres, more preferably less than about 5 square millimetres.

The ratio of the maximum flow area of each of the first and second airflow apertures to the surface area of the downstream end of the blocking element is preferably at least about 0.06, more preferably at least about 0.08, more preferably at least about 0.1. The ratio of the maximum flow area of each of the first and second airflow apertures to the surface area of the downstream end of the blocking element is preferably less than about 0.2, more preferably less than about 0.16, more preferably less than about 0.14.

The blocking element may comprise a first blocking portion positioned between a first end of the first airflow aperture and a second end of the second airflow aperture. The blocking element may comprise a second blocking portion positioned between a second end of the first airflow aperture and a first end of the second airflow aperture. The first blocking portion is configured to obstruct the first air outlet when the cartridge is in the first position and the second blocking portion is configured to obstruct the second air outlet when the cartridge is in the first position.

The cartridge assembly may further comprise a seal extending across the upstream end of the cartridge. Preferably, the seal is secured to the cartridge about a periphery of the seal to seal the first air inlet and the second air inlet. In embodiments in which the cartridge comprises a cartridge housing and a cartridge body, the seal may be secured to at least one of the cartridge housing and the cartridge body. The seal may be secured to the cartridge by at least one of an adhesive and a weld, such as an ultrasonic weld. The seal is preferably formed from a sheet material. The sheet material may comprise at least one of a polymeric film and a metallic foil.

The seal may be a frangible seal configured to be pierced by a piercing element on an aerosol-generating device.

The seal may be a removable seal configured to be removed by a user before using the cartridge assembly. The removable seal may comprise a pull tab to facilitate removal of the seal by a user.

The first air outlet may comprise a single first air outlet aperture. The first air outlet may comprise a plurality of first air outlet apertures, each first air outlet aperture in fluid communication with the downstream end of the first compartment. Each first air outlet aperture may have a minimum cross-sectional area, the minimum cross-sectional area being the flow area of the first air outlet aperture. The total flow area of the first air outlet is the sum of the flow areas of the one or more first air outlet apertures.

The second air outlet may comprise a single second air outlet aperture. The second air outlet may comprise a plurality of second air outlet apertures, each second air outlet aperture in fluid communication with the downstream end of the second compartment. Each second air outlet aperture may have a minimum cross-sectional area, the minimum cross-sectional area being the flow area of the second air outlet aperture. The total flow area of the second air outlet is the sum of the flow areas of the one or more second air outlet apertures.

The total flow area of the first air outlet may be the same as the total flow area of the second air outlet. The total flow area of the first air outlet may be different to the total flow area of the second air outlet. Different total flow areas may be selected to provide different flow rates of air through each of the first compartment and the second compartment. In embodiments in which the cartridge comprises a source of a first volatile compound positioned within the first compartment and a source of a second volatile compound in the second compartment, providing different flow rates through the first and second compartments may account for a difference between a vapour pressure of the first volatile compound and a vapour pressure of the second volatile compound at the same temperature. In embodiments in which the first and second volatile compounds undergo a chemical reaction with each other to form a reaction product for delivery to a user, providing different flow rates through the first and second compartments may provide a desired reaction stoichiometry between the first and second volatile compounds downstream of the cartridge.

The optional and preferred features described herein with respect to the first and second air outlets may be applied equally to the first and second air inlets. That is, each of the first and second air inlets may comprise one or more air inlet apertures. The total flow area of the first air inlet may be the same as the total flow area of the second air inlet. The total flow area of the first air inlet may be different to the total flow area of the second air inlet.

The cartridge assembly may comprise a first indicium on the mouthpiece housing and a second indicium on the cartridge. Preferably, the indicia are configured such that the position of the first indicium with respect to the second indicium provides a visual indication to a user of the rotational orientation of the cartridge with respect to the mouthpiece housing. Advantageously, providing a visual indication of a rotational orientation of the cartridge allows a user to determine whether the first and second air outlets are obstructed by the blocking element or unobstructed and in fluid communication with the mouthpiece cavity.

The cartridge assembly may comprise a nicotine source positioned within the first compartment. The cartridge assembly may comprise an acid source positioned within the second compartment.

As used herein with reference to the invention, the term "nicotine", is used to describe nicotine, nicotine base or a nicotine salt.

The nicotine source may comprise a first carrier material impregnated with between about 1 milligram and about 50 milligrams of nicotine. The nicotine source may comprise a first carrier material impregnated with between about 1 milligram and about 40 milligrams of nicotine. Preferably, the nicotine source comprises a first carrier material impregnated with between about 3 milligrams and about 30 milligrams of nicotine. More preferably, the nicotine source comprises a first carrier material impregnated with between about 6 milligrams and about 20 milligrams of nicotine. Most preferably, the nicotine source comprises a first carrier material impregnated with between about 8 milligrams and about 18 milligrams of nicotine.

In embodiments in which the first carrier material is impregnated with nicotine base or a nicotine salt, the amounts of nicotine recited herein are the amount of nicotine base or amount of ionised nicotine, respectively.

The first carrier material may be impregnated with liquid nicotine or a solution of nicotine in an aqueous or non-aqueous solvent.

The first carrier material may be impregnated with natural nicotine or synthetic nicotine.

The acid source may comprise an organic acid or an inorganic acid.

Preferably, the acid source comprises an organic acid, more preferably a carboxylic acid, most preferably an alpha-keto or 2-oxo acid or lactic acid.

Advantageously, the acid source comprises an acid selected from the group consisting of 3-methyl-2-oxopentanoic acid, pyruvic acid, 2-oxopentanoic acid, 4-methyl-2-oxopentanoic acid, 3-methyl-2-oxobutanoic acid, 2-oxooctanoic acid, lactic acid and combinations thereof. Advantageously, the acid source comprises pyruvic acid or lactic acid. More advantageously, the acid source comprises lactic acid.

Advantageously, the acid source comprises a second carrier material impregnated with acid.

The first carrier material and the second carrier material may be the same or different.

Advantageously, the first carrier material and the second carrier material have a density of between about 0.1 grams/cubic centimetre and about 0.3 grams/cubic centimetre.

Advantageously, the first carrier material and the second carrier material have a porosity of between about 15 percent and about 55 percent.

The first carrier material and the second carrier material may comprise one or more of glass, cellulose, ceramic, stainless steel, aluminium, polyethylene (PE), polypropylene, polyethylene terephthalate (PET), poly(cyclohexanedimethylene terephthalate) (PCT), polybutylene terephthalate (PBT), polytetrafluoroethylene (PTFE), expanded polytetrafluoroethylene (ePTFE), and BAREX®.

The first carrier material acts as a reservoir for the nicotine.

Advantageously, the first carrier material is chemically inert with respect to nicotine.

The first carrier material may have any suitable shape and size. For example, the first carrier material may be in the form of a sheet or plug.

Advantageously, the shape and size of the first carrier material is similar to the shape and size of the first compartment of the cartridge.

The shape, size, density and porosity of the first carrier material may be chosen to allow the first carrier material to be impregnated with a desired amount of nicotine.

Advantageously, the first compartment of the cartridge may further comprise a flavourant. Suitable flavourants include, but are not limited to, menthol.

Advantageously, the first carrier material may be impregnated with between about 3 milligrams and about 12 milligrams of flavourant.

The second carrier material acts as a reservoir for the acid.

Advantageously, the second carrier material is chemically inert with respect to the acid.

The second carrier material may have any suitable shape and size. For example, the second carrier material may be in the form of a sheet or plug.

Advantageously, the shape and size of the second carrier material is similar to the shape and size of the second compartment of the cartridge.

The shape, size, density and porosity of the second carrier material may be chosen to allow the second carrier material to be impregnated with a desired amount of acid.

Advantageously, acid source is a lactic acid source comprising a second carrier material impregnated with between about 2 milligrams and about 60 milligrams of lactic acid.

Preferably, the lactic acid source comprises a second carrier material impregnated with between about 5 milligrams and about 50 milligrams of lactic acid. More preferably, the lactic acid source comprises a second carrier material impregnated with between about 8 milligrams and about 40 milligrams of lactic acid. Most preferably, the lactic acid source comprises a second carrier material impregnated with between about 10 milligrams and about 30 milligrams of lactic acid.

The shape and dimensions of the first compartment of the cartridge may be chosen to allow a desired amount of nicotine to be housed in the cartridge.

The shape and dimensions of the second compartment of the cartridge may be chosen to allow a desired amount of acid to be housed in the cartridge.

The ratio of nicotine and acid required to achieve an appropriate reaction stoichiometry may be controlled and balanced through variation of the volume of the first compartment relative to the volume of the second compartment.

In embodiments in which the cartridge assembly comprises a nicotine source positioned within the first compartment and an acid source positioned within the second compartment, nicotine vapour released from the nicotine source in the first compartment of the cartridge and acid vapour released from the acid source in the second compartment of the cartridge may react with one another in the gas phase in the mouthpiece cavity to form an aerosol of nicotine salt particles.

The first compartment of the cartridge may be coated with one or more nicotine-resistant materials and the second compartment of the cartridge may be coated with one or more acid-resistant materials.

Examples of suitable nicotine-resistant materials and acid-resistant materials include, but are not limited to, polyethylene (PE), polypropylene (PP), polystyrene (PS), fluorinated ethylene propylene (FEP), polytetrafluoroethylene (PTFE), epoxy resins, polyurethane resins, vinyl resins and combinations thereof.

Use of one or more nicotine-resistant materials to one or both of form the cartridge and coat the interior of the first compartment of the cartridge may advantageously enhance the shelf life of the cartridge assembly.

Use of one or more acid-resistant materials to one or both of form the cartridge and coat the interior of the second compartment of the cartridge may advantageously enhance the shelf life of the cartridge assembly.

The cartridge assembly may comprise one or more aerosol-modifying agents positioned within the mouthpiece cavity. For example, mouthpiece cavity may contain one or more sorbents, one or more flavourants, one or more chemesthetic agents or a combination thereof.

The cartridge may comprise a third compartment for receiving a heating element of an aerosol-generating device. Preferably, the third compartment is positioned between the first compartment and the second compartment. That is, the first compartment and the second compartment are disposed on either side of the third compartment. Preferably, the third compartment comprises a compartment opening at the upstream end of the cartridge. In use, a heating element of an aerosol-generating device is received within the third compartment to heat the first compartment and the second compartment.

The cartridge may comprise a susceptor for inductively heating the first compartment and the second compartment. In such embodiments, the susceptor is advantageously located between the first compartment and the second compartment. That is, the first compartment and the second compartment are disposed on either side of the susceptor.

In use, heating the first compartment and the second compartment of the cartridge to a temperature above ambient temperature advantageously enables control of the vapour concentrations of volatile compounds stored within the first and second compartments. For example, in embodiments in which the cartridge assembly comprises a nicotine source positioned within the first compartment and an acid source positioned within the second compartment, heating the first and second compartments enables the vapour pressure of nicotine in the first compartment and the vapour pressure of acid in the second compartment to be controlled and balanced proportionally to yield an efficient reaction stoichiometry between the nicotine and the acid. Advantageously, this may improve the efficiency of the formation of nicotine salt particles and the consistency of delivery to a user. Advantageously, it may also reduce the delivery of unreacted nicotine and unreacted acid to a user.

Each of the cartridge, the blocking element, and the mouthpiece housing may be formed from any suitable material or combination of materials. Suitable materials include, but are not limited to, aluminium, polyether ether ketone (PEEK), polyimides, such as Kapton®, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), epoxy resins, polyurethane resins, vinyl resins, liquid crystal polymers (LCP) and modified LCPs, such as LCPs with graphite or glass fibres.

The cartridge, the blocking element, and the mouthpiece housing may be formed from the same or different materials. Preferably, the mouthpiece housing is formed from at least one of polyethylene (PE) and polypropylene (PP). Preferably, the blocking element is formed from polyethylene (PE). In embodiments in which the cartridge comprises a cartridge housing, preferably the cartridge housing is formed from polypropylene (PP). In embodiments in which the cartridge comprises a cartridge body, preferably the cartridge is formed from at least one of polyether ether ketone (PEEK) and liquid crystal polymers (LCP).

The cartridge assembly may simulate the shape and dimensions of a combustible smoking article, such as a cigarette, a cigar, or a cigarillo. Advantageously, in such embodiments the cartridge assembly may simulate the shape and dimensions of a cigarette.

The cartridge assembly may have a length of between about 20 millimetres and about 60 millimetres, preferably between about 30 and about 50 millimetres, more preferably between about 35 millimetres and about 45 millimetres.

The cartridge assembly may have a diameter of between about 5 millimetres and about 10 millimetres, preferably between about 6 millimetres and about 9 millimetres, more preferably between about 7 millimetres and about 8 millimetres.

According to a second aspect of the present invention there is provided an aerosol-generating system comprising an aerosol-generating device and a cartridge assembly according to the first aspect of the present invention, in accordance with any of the embodiments described herein. The aerosol-generating device comprises a device cavity configured to receive an upstream end of the cartridge assembly and a heater for heating the first compartment and the second compartment of the cartridge of the cartridge assembly.

In those embodiments in which the cartridge comprises a third compartment for receiving a heating element, the heater of the aerosol-generating device advantageously comprises a heating element positioned within the device cavity and configured to be received within the third compartment of the cartridge when the upstream end of the cartridge assembly is received within the device cavity. The heating element may be a resistive heating element. In use, the heating element is received within the third compartment and heats the first compartment and the second compartment.

In those embodiments in which the cartridge comprises a susceptor positioned between the first compartment and the second compartment, the heater of the aerosol-generating device advantageously comprises an inductive heater surrounding at least a portion of the device cavity. In use, the inductive heater inductively heats the susceptor, which heats the first compartment and the second compartment.

The aerosol-generating system may be configured so that, in use, the heater heats the first compartment and the second compartment of the cartridge to a temperature of below about 250 degrees Celsius. Preferably, the aerosol-generating system is configured so that the heater heats the first compartment and the second compartment of the cartridge to a temperature of between about 80 degrees Celsius and about 150 degrees Celsius.

Preferably, the aerosol-generating system is configured so that, in use, the heater heats the first compartment and the second compartment of the cartridge to substantially the same temperature.

The aerosol-generating device may further comprise a power supply for supplying power to the heater and a controller configured to control a supply of power from the power supply to the heater.

The aerosol-generating device may comprise one or more temperature sensors configured to sense the temperature of at least one of the heater, the first compartment, and the second compartment. In such embodiments, the controller may be configured to control a supply of power to the heater based on a sensed temperature.

For the avoidance of doubt, features described above in relation to one aspect of the invention may also be applicable to other aspects of the invention. In particular, features described above in relation to the cartridge assembly of the invention may also relate, where appropriate, to the aerosol-generating systems of the invention, and *vice versa.*

Embodiments of the invention will now be described, by way of example only, with reference to the accompanying drawings, in which:
Figure 1 shows a perspective view of a cartridge assembly according to an embodiment of the present invention;
Figure 2 shows an exploded perspective view of the cartridge assembly of Figure 1;
Figure 3 shows perspective views of the cartridge housing and the cartridge body of Figure 2;
Figure 4 shows a view of the upstream end of the cartridge body of Figure 2;
Figure 5 shows an exploded view of the downstream end of the cartridge body of Figure 2;
Figure 6 shows a perspective view of the cartridge housing of Figure 2; and
Figure 7 shows a view of the downstream end of the blocking element of Figure 2.

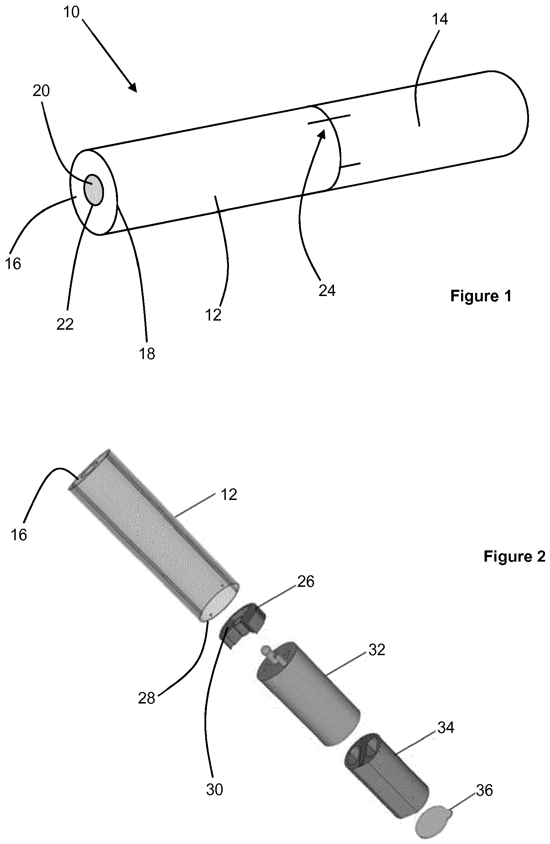

Figure 1 shows a cartridge assembly 10 according to an embodiment of the present invention. The cartridge assembly comprises a mouthpiece housing 12 and a cartridge 14 configured for rotation with respect to the mouthpiece housing 12. The mouthpiece housing 12 comprises a downstream end wall 16 extending across a downstream end 18 of the mouthpiece housing 12. The mouthpiece housing 12 defines a mouthpiece cavity 20 and comprises a mouthpiece housing air outlet 22 extending through the downstream end wall 16 and in fluid communication with the mouthpiece cavity 20. A plurality of indicia 24 on the outside of the mouthpiece housing 12 and the cartridge 14 indicate the rotational orientation of the cartridge 14 with respect to the mouthpiece housing 12.

Figure 2 shows an exploded perspective view of the cartridge assembly 10 of Figure 1. As shown in Figure 2, the cartridge assembly 10 further comprises a blocking element 26 that is secured by an interference fit in the downstream end 28 of the mouthpiece housing 12. The mouthpiece cavity 20 extends between a downstream end 30 of the blocking element 26 and the downstream end wall 16 of the mouthpiece housing 12.

The cartridge 14 comprises a cartridge housing 32 and a cartridge body 34 that is secured within the cartridge housing 32 by an interference fit. A removable seal 36 is secured to the upstream end of the cartridge 14, the removable seal 36 configured to be removed by a user prior to using the cartridge assembly 10.

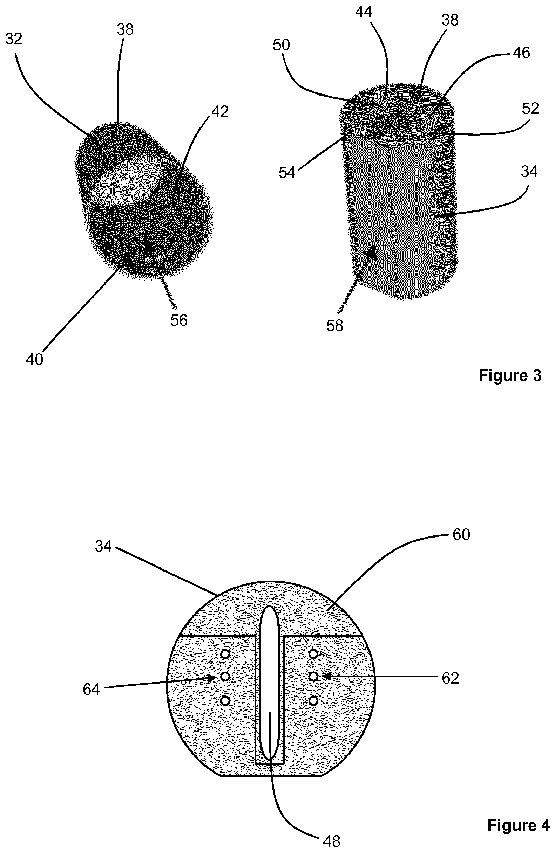

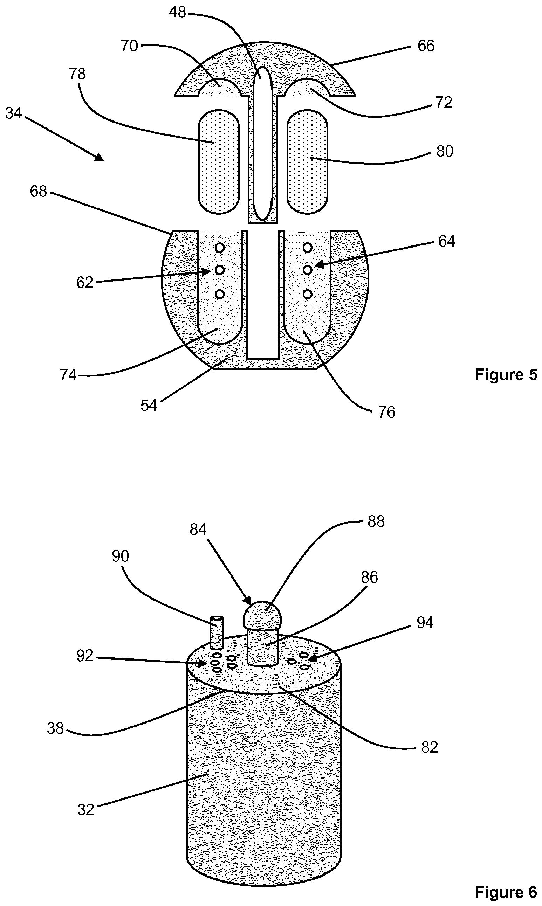

Figure 3 shows the cartridge housing 32 and the cartridge body 34 in further detail. The cartridge housing 32 comprises a downstream end 38 and an upstream end 40, the cartridge housing 32 defining a cavity 42 for receiving the cartridge body 34. The upstream end 40 of the cartridge housing 32 is open for insertion of the cartridge body 34 into the cavity 42 during manufacture of the cartridge 14. The cartridge body 34 comprises a first compartment 44, a second compartment 46 and a third compartment 48 positioned between the first and second compartments 44, 46. The first and second compartments 44, 46 are open at their downstream ends 50, 52 at the downstream end 54 of the cartridge body 34. The cartridge housing 32 and the cartridge body 34 comprise mating portions 56, 58 so that the cartridge body 34 can be inserted into the cartridge housing 32 in only a single orientation. This ensures correct alignment between the downstream ends of the first and second compartments 44, 46 and first and second air outlets provided on the downstream end 38 of the cartridge housing 32, as will be further described with reference to Figure 6.

Figure 4 shows the upstream end 60 of the cartridge body 34, which comprises a first air inlet 62 comprising a plurality of first air inlet apertures in fluid communication with the first compartment 44 and a second air inlet 64 comprising a plurality of second air inlet apertures in fluid communication with the second compartment 46. The third compartment 48 is open at the upstream end 60 of the cartridge body 34 to receive a heater of an aerosol-generating device for heating the first and second compartments 44, 46. In an alternative embodiment the cartridge body 34 may comprise a susceptor positioned within the third compartment 48 for use with an aerosol-generating device comprising an inductive heater.

Figure 5 shows an exploded view of the downstream end 54 of the cartridge body 34. The cartridge body 34 comprises a first part 66 and a second part 68 that are secured together by an interference fit to form the cartridge body 34. The first part 66 defines the third compartment 48, a first side 70 of the first compartment 44, and a first side 72 of the second compartment 46. The second part 68 defines a second side 74 of the first compartment 44, a second side 76 of the second compartment 46, the first air inlet 62 and the second air inlet 64. During manufacture and assembly of the cartridge body 34, a nicotine source 78 is inserted into the second side 74 of the first compartment 44 and an acid source 80 is inserted into the second side 76 of the second compartment 46, prior to the first and second parts 66, 68 being secured together.

As shown in Figure 6, the cartridge housing 32 comprises a downstream end wall 82 extending across a downstream end 38 of the cartridge housing 32. The cartridge housing 32 further comprises a shaft 84 extending from the downstream end wall 82, the shaft 84 comprising a journal portion 86 and a retaining portion 88. The retaining portion 88 has a rounded shape and has a maximum diameter that is larger than the diameter of the journal portion 86. The cartridge housing 32 further comprises a guiding pin 90 extending from the downstream end wall 82, a first air outlet 92 and a second air outlet 94. When the cartridge body 34 is received within the cartridge housing 32 the first air outlet 92 is in fluid communication with the downstream end 50 of the first compartment 44 and the second air outlet 94 is in fluid communication with the downstream end 52 of the second compartment 46.

The first air outlet 92 comprise a different number of first air outlet apertures compared to the number of second air outlet apertures forming the second air outlet 94. The different number of air outlet apertures provides different flow rates of air through the first and second compartments 44, 46 during use of the cartridge assembly 10 to account for the different vapour pressures of the nicotine source 78 and the acid source 80. Therefore, the different flow rates of air through the first and second compartments 44, 46 maintains a required reaction stoichiometry between nicotine vapour and acid vapour downstream of the cartridge 14 in the mouthpiece cavity 20.

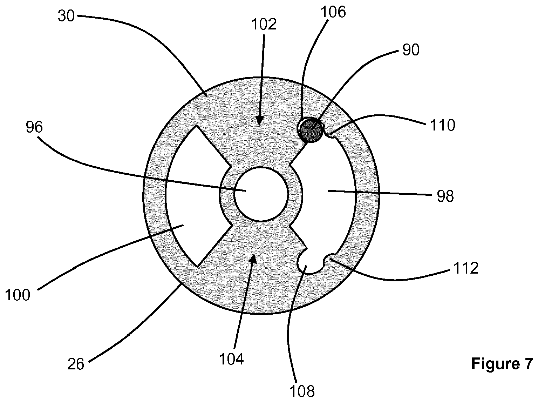

Figure 7 shows the downstream end 30 of the blocking element 26. The blocking element 26 comprises an aperture 96 for receiving the journal portion 86 of the shaft 84 on the cartridge housing 32. The shaft 84 has been omitted from Figure 7 to show the aperture 96. During assembly of the cartridge assembly 10, the aperture 96 is elastically deformed to accommodate the retaining portion 88 of the shaft 84 as the retaining portion 88 is pushed through the aperture 96. Once the retaining portion 88 has been pushed fully through the aperture 96, the step change in diameter between the retaining portion 88 and the journal portion 86 prevents the retaining portion 88 being pulled back through the aperture 96. The diameter of the journal portion 86 is slightly smaller than the diameter of the aperture 96 so that the journal portion 86 may rotate freely within the aperture 96, which allows rotation of the cartridge 14 with respect to the blocking element 26. When the journal portion 86 is positioned within the aperture 96, the downstream end wall 82 of the cartridge housing 32 abuts the upstream end of the blocking element 26.

The blocking element 26 further comprises a first airflow aperture 98 and a diametrically opposed second airflow aperture 100, each airflow aperture having a quarter-circular shape. A first blocking portion 102 extends between a first end of the first airflow aperture 98 and a second end of the second airflow aperture 100. A second blocking portion 104 extends between a second end of the first airflow aperture 98 and a first end of the second airflow aperture 100.

The first airflow aperture 98 also forms a guide opening in which the guiding pin 90 of the cartridge body 32 is received. The blocking element 26 comprises a first recess 106 at the first end of the first airflow aperture 98 for receiving the guiding pin 90 when the cartridge 14 is in a first position with respect to the blocking element 26. The blocking element 26 comprises a second recess 108 at the second end of the first airflow aperture 98 for receiving the guiding pin 90 when the cartridge 14 is in a second position with respect to the blocking element 26.

When the cartridge 14 is in the first position, the first blocking portion 102 obstructs the first air outlet 92 on the cartridge body 32 and the second blocking portion 104 obstructs the second air outlet 94 on the cartridge body 32. A first detent 110 on the blocking element 26 increases the force required to move the guiding pin 90 out of the first recess 106, which reduces the risk of the cartridge 14 being accidentally rotated out of the first position.

When the cartridge 14 is in the second position, the first air outlet 92 on the cartridge body 32 is in fluid communication with the mouthpiece cavity 20 via the first airflow aperture 98 and the second air outlet 94 on the cartridge body 32 is in fluid communication with the mouthpiece cavity 20 via the second airflow aperture 100. A second detent 112 on the blocking element 26 increases the force required to move the guiding pin 90 into the second recess 108, which provides a user with a tactile confirmation when the cartridge 14 has been fully rotated into the second position. The second detent 112 also retains the guiding pin 90 in the second recess 108 to retain the cartridge 14 in the second position during use of the cartridge assembly 10.

During use of the cartridge assembly 10, a user removes the removable seal 36 from the upstream end of the cartridge 14 and rotates the cartridge 14 into the second position so that the first and second air outlets 92, 94 are in fluid communication with the mouthpiece cavity 20. The cartridge assembly 10 is combined with an aerosol-generating device to form an aerosol-generating system, the cartridge assembly 10 receiving a heater of the aerosol-generating device into the third compartment 48 to heat the first and second compartments 44, 46. When a user draws on the downstream end 16 of the mouthpiece housing 12, air is drawing into the cartridge assembly 10 through the first and second air inlets 62, 64. Air flows from the first and second air inlets 62, 64 through the first and second compartments 44, 46 where nicotine vapour and acid vapour are entrained in the airflow. The nicotine and acid vapours flow through the first and second air outlets 92, 94 into the mouthpiece cavity 20 via the first and second airflow apertures 98, 100 of the blocking element 26. In the mouthpiece cavity 20 the nicotine and acid vapours react in the gas phase to form an aerosol of nicotine salt particles which is delivered to the user through the mouthpiece housing air outlet 22.

## Claims

1. A cartridge assembly (10) for use in an aerosol-generating system, the cartridge assembly (10) comprising:
a mouthpiece housing (12) having an upstream end and a downstream end, the mouthpiece housing (12) defining a mouthpiece cavity (20) extending between the upstream end of the mouthpiece housing (12) and the downstream end of the mouthpiece housing (12);
a blocking element (26) secured within the mouthpiece cavity (20) and comprising an upstream end and a downstream end; and
a cartridge (14) positioned at the upstream end of the mouthpiece housing (12), the cartridge (14) comprising:
an upstream end and a downstream end;
a first compartment (44) having a first air inlet (62) at the upstream end of the cartridge (14) and a first air outlet (92) at the downstream end of the cartridge (14); and
a second compartment (46) having a second air inlet (64) at the upstream end of the cartridge (14) and a second air outlet (94) at the downstream end of the cartridge (14);
wherein the downstream end of the cartridge (14) abuts the upstream end of the blocking element (26);
wherein the cartridge (14) is rotatable with respect to the blocking element (26) from a first position to a second position; and
wherein the blocking element (26) is shaped so that the upstream end of the blocking element (26) obstructs the first air outlet (92) and the second air outlet (94) when the cartridge (14) is in the first position, and the first air outlet (92) and the second air outlet (94) are both in fluid communication with the mouthpiece cavity (20) when the cartridge (14) is in the second position.

2. A cartridge assembly (10) according to claim 1, wherein one of the blocking element (26) and the cartridge (14) comprises an aperture (96) and wherein the other of the blocking element (26) and the cartridge (14) comprises a shaft (84), wherein at least a portion of the shaft (84) is positioned within the aperture (96).

3. A cartridge assembly (10) according to claim 2, wherein the aperture (96) extends through the blocking element (26) between the upstream end of the blocking element (26) and the downstream end of the blocking element (26), and wherein the shaft (84) extends from the downstream end of the cartridge (14).

4. A cartridge assembly (10) according to claim 2 or 3, wherein the shaft (84) comprises a journal portion (86) positioned within the aperture (96) and a retaining portion (88) at an end of the journal portion (86), wherein the retaining portion (88) is positioned outside of the aperture (96) and has a diameter that is larger than a diameter of the aperture (96).

5. A cartridge assembly (10) according to any preceding claim, wherein the cartridge (14) comprises a guiding pin (90) extending from the downstream end of the cartridge (14), wherein the guiding pin (90) abuts a first portion of the blocking element (26) when the cartridge (14) is in the first position, and wherein the guiding pin (90) abuts a second portion of the blocking element (26) when the cartridge (14) is in the second position.

6. A cartridge assembly (10) according to any preceding claim, wherein the cartridge (14) comprises:
a cartridge housing (32) positioned at the upstream end of the mouthpiece housing (12) and rotatable with respect to the blocking element (26) from the first position to the second position; and
a cartridge body (34) secured within the cartridge housing (32), the cartridge body (34) defining the first compartment (44) and the second compartment (46).

7. A cartridge assembly (10) according to any preceding claim, wherein the blocking element (26) is spaced apart from the downstream end of the mouthpiece housing (12), and wherein a portion of the mouthpiece cavity (20) between the downstream end of the blocking element (26) and the downstream end of the mouthpiece housing (12) forms a mixing chamber.

8. A cartridge assembly (10) according to claim 7, wherein the mouthpiece housing (12) comprises a ventilation air inlet positioned between the blocking element (26) and the downstream end of the mouthpiece housing (12), the ventilation air inlet extending through the mouthpiece housing (12) and providing fluid communication between the exterior of the mouthpiece housing (12) and the mixing chamber.

9. A cartridge assembly (10) according to any preceding claim, wherein the blocking element (26) comprises a first airflow aperture (98) configured to overlie the first air outlet (92) when the cartridge (14) is in the second position and a second airflow aperture (100) configured to overlie the second air outlet (94) when the cartridge (14) is in the second position.

10. A cartridge assembly (10) according to claim 9, wherein the blocking element (26) has a circular shape and wherein the first airflow aperture (98) and the second airflow aperture (100) each have an arcuate shape.

11. A cartridge assembly (10) according to claim 10, wherein the first airflow aperture (98) and the second airflow aperture (100) are diametrically opposed.

12. A cartridge assembly (10) according to claim 10 or 11, wherein the first airflow aperture (98) and the second airflow aperture (100) each extend along a quarter-circular arc length.

13. A cartridge assembly (10) according to any of claims 9 to 12, wherein the blocking element (26) comprises a first blocking portion (102) positioned between a first end of the first airflow aperture (98) and a second end of the second airflow aperture (100), wherein the blocking element (26) comprises a second blocking portion (104) positioned between a second end of the first airflow aperture (98) and a first end of the second airflow aperture (100), wherein the first blocking portion (102) is configured to obstruct the first air outlet (92) when the cartridge (14) is in the first position, and wherein the second blocking portion (104) is configured to obstruct the second air outlet (94) when the cartridge (14) is in the first position.

14. A cartridge assembly (10) according to any preceding claim, wherein the mouthpiece housing (12) comprises a downstream wall portion extending across the downstream end of the mouthpiece cavity (20), the mouthpiece housing (12) comprising a mouthpiece housing air outlet (22) extending through the downstream wall portion.

15. A cartridge assembly (10) according to any preceding claim, further comprising a seal (36) extending across the upstream end of the cartridge (14).

16. A cartridge assembly (10) according to any preceding claim, wherein the cartridge (14) further comprises a nicotine source (78) positioned within the first compartment (44) and an acid source (80) positioned within the second compartment (46).

17. A cartridge assembly (10) according to any preceding claim, wherein the cartridge (14) comprises a third compartment (48) for receiving a heating element of an aerosol-generating device, wherein the third compartment (48) comprises a compartment opening at the upstream end of the cartridge (14), and wherein the third compartment is positioned between the first compartment (44) and the second compartment (46).

18. A cartridge assembly (10) according to any of claims 1 to 16, wherein the cartridge (14) comprises a susceptor positioned between the first compartment (44) and the second compartment (46).

19. An aerosol-generating system comprising:
a cartridge assembly (10) according to any of claims 1 to 16; and
an aerosol-generating device comprising a device cavity configured to receive an upstream end of the cartridge assembly (10) and a heater for heating the first compartment (44) and the second compartment (46) of the cartridge (14) of the cartridge assembly (10).

20. An aerosol-generating system according to claim 19, wherein the heater comprises a heating element positioned within the device cavity, wherein the cartridge (14) comprises a third compartment (48) for receiving the heating element, wherein the third compartment (48) comprises a compartment opening at the upstream end of the cartridge (14), and wherein the third compartment (48) is positioned between the first compartment (44) and the second compartment (46).

21. An aerosol-generating system according to claim 19, wherein the heater comprises an inductive heater surrounding at least a portion of the device cavity, and wherein the cartridge (14) comprises a susceptor positioned between the first compartment (44) and the second compartment (46).

## Patentansprüche

1. Patronenanordnung (10) zum Gebrauch in einem Aerosolerzeugungssystem, wobei die Patronenanordnung (10) aufweist:
ein Mundstückgehäuse (12) mit einem zuströmseitigen Ende und einem nachgeschalteten Ende, wobei das Mundstückgehäuse (12) einen Mundstückhohlraum (20) definiert, der sich zwischen dem zuströmseitigen Ende des Mundstückgehäuses (12) und dem nachgeschalteten Ende des Mundstückgehäuses (12) erstreckt;
ein Sperrelement (26), das innerhalb des Mundstückhohlraums (20) befestigt ist und ein zuströmseitiges Ende und ein nachgeschaltetes Ende aufweist; und
eine Patrone (14), die am zuströmseitigen Ende des Mundstückgehäuses (12) angeordnet ist, wobei die Patrone (14) Folgendes aufweist:
ein zuströmseitiges Ende und ein nachgeschaltetes Ende;
eine erste Kammer (44) mit einem ersten Lufteinlass (62) an dem zuströmseitigen Ende der Patrone (14) und einem ersten Luftauslass (92) an dem nachgeschalteten Ende der Patrone (14); und
eine zweite Kammer (46) mit einem zweiten Lufteinlass (64) an dem zuströmseitigen Ende der Patrone (14) und einem zweiten Luftauslass (94) an dem nachgeschalteten Ende der Patrone (14);
wobei das nachgeschaltete Ende der Patrone (14) an dem zuströmseitigen Ende des Sperrelements (26) anliegt;
wobei die Patrone (14) in Bezug auf das Sperrelement (26) von einer ersten Stellung in eine zweite Stellung drehbar ist; und
wobei das Sperrelement (26) so geformt ist, dass das zuströmseitige Ende des Sperrelements (26) den ersten Luftauslass (92) und den zweiten Luftauslass (94) versperrt, wenn sich die Patrone (14) in der ersten Stellung befindet, und der erste Luftauslass (92) und der zweite Luftauslass (94) beide in Fluidverbindung mit dem Mundstückhohlraum (20) stehen, wenn sich die Patrone (14) in der zweiten Stellung befindet.

2. Patronenanordnung (10) nach Anspruch 1, wobei das Sperrelement (26) oder die Patrone (14) eine Aussparung (96) aufweist und wobei das jeweils andere Sperrelement (26) und die jeweils andere Patrone (14) eine Welle (84) aufweist, wobei zumindest ein Abschnitt der Welle (84) innerhalb der Aussparung (96) angeordnet ist.

3. Patronenanordnung (10) nach Anspruch 2, wobei die Aussparung (96) durch das Sperrelement (26) zwischen dem zuströmseitigen Ende des Sperrelements (26) und dem nachgeschalteten Ende des Sperrelements (26) verläuft, und wobei sich die Welle (84) von dem zuströmseitigen Ende der Patrone (14) erstreckt.

4. Patronenanordnung (10) nach Anspruch 2 oder 3, wobei die Welle (84) einen Lagerzapfenabschnitt (86), der innerhalb der Aussparung (96) angeordnet ist, und einen Rückhalteabschnitt (88) an einem Ende des Lagerzapfenabschnitts (86) aufweist, wobei der Rückhalteabschnitt (88) außerhalb der Aussparung (96) angeordnet ist und einen Durchmesser hat, der größer als ein Durchmesser der Aussparung (96) ist.

5. Patronenanordnung (10) nach einem der vorhergehenden Ansprüche, wobei die Patrone (14) einen Führungsstift (90) aufweist, der sich von dem nachgeschalteten Ende der Patrone (14) erstreckt, wobei der Führungsstift (90) an einem ersten Abschnitt des Sperrelements (26) anliegt, wenn sich die Patrone (14) in der ersten Stellung befindet, und wobei der Führungsstift (90) an einem zweiten Abschnitt des Sperrelements (26) anliegt, wenn sich die Patrone (14) in der zweiten Stellung befindet.

6. Patronenanordnung (10) nach einem der vorhergehenden Ansprüche, wobei die Patrone (14) aufweist:
ein Patronengehäuse (32), das an dem zuströmseitigen Ende des Mundstückgehäuses (12) angeordnet und in Bezug auf das Sperrelement (26) von der ersten Stellung in die zweite Stellung drehbar ist; und
einen Patronenkörper (34), der innerhalb des Patronengehäuses (32) befestigt ist, wobei der Patronenkörper (34) die erste Kammer (44) und die zweite Kammer (46) definiert.

7. Patronenanordnung (10) nach einem der vorstehenden Ansprüche, wobei das Sperrelement (26) von dem nachgeschalteten Ende des Mundstückgehäuses (12) beabstandet ist, und wobei ein Abschnitt des Mundstückhohlraums (20) zwischen dem nachgeschalteten Ende des Sperrelements (26) und dem nachgeschalteten Ende des Mundstückgehäuses (12) eine Mischkammer ausbildet.

8. Patronenanordnung (10) nach Anspruch 7, wobei das Mundstückgehäuse (12) einen Belüftungslufteinlass aufweist, der zwischen dem Sperrelement (26) und dem nachgeschalteten Ende des Mundstückgehäuses (12) angeordnet ist, wobei der Belüftungslufteinlass durch das Mundstückgehäuse (12) verläuft und eine Fluidverbindung zwischen dem Äußeren des Mundstückgehäuses (12) und der Mischkammer vorsieht.

9. Patronenanordnung (10) nach einem der vorhergehenden Ansprüche, wobei das Sperrelement (26) eine erste Luftstromaussparung (98), die so ausgelegt ist, dass sie über dem ersten Luftauslass (92) liegt, wenn sich die Patrone (14) in der zweiten Position befindet, und eine zweite Luftstromaussparung (100), die so ausgelegt ist, dass sie über dem zweiten Luftauslass (94) liegt, wenn sich die Patrone (14) in der zweiten Position befindet, aufweist.

10. Patronenanordnung (10) nach Anspruch 9, wobei das Sperrelement (26) eine kreisförmige Form hat und wobei die erste Luftstromaussparung (98) und die zweite Luftstromaussparung (100) jeweils eine bogenförmige Form haben.

11. Patronenanordnung (10) nach Anspruch 10, wobei die erste Luftstromaussparung (98) und die zweite Luftstromaussparung (100) diametral gegenüberliegen.

12. Patronenanordnung (10) nach Anspruch 10 oder 11, wobei sich die erste Luftstromaussparung (98) und die zweite Luftstromaussparung (100) jeweils entlang einer viertelkreisförmigen Bogenlänge erstrecken.

13. Patronenanordnung (10) nach einem der Ansprüche 9 bis 12, wobei das Sperrelement (26) einen ersten Sperrabschnitt (102) aufweist, der zwischen einem ersten Ende der ersten Luftstromaussparung (98) und einem zweiten Ende der zweiten Luftstromaussparung (100) angeordnet ist, wobei das Sperrelement (26) einen zweiten Sperrabschnitt (104) aufweist, der zwischen einem zweiten Ende der ersten Luftstromaussparung (98) und einem ersten Ende der zweiten Luftstromaussparung (100) angeordnet ist, wobei der erste Sperrabschnitt (102) ausgelegt ist, den ersten Luftauslass (92) zu versperren, wenn sich die Patrone (14) in der ersten Stellung befindet, und wobei der zweite Sperrabschnitt (104) ausgelegt ist, den zweiten Luftauslass (94) zu versperren, wenn sich die Patrone (14) in der ersten Stellung befindet.

14. Patronenanordnung (10) nach einem der vorhergehenden Ansprüche, wobei das Mundstückgehäuse (12) einen nachgeschalteten Wandabschnitt aufweist, der sich über das nachgeschaltete Ende des Mundstückhohlraums (20) erstreckt, wobei das Mundstückgehäuse (12) einen Mundstückgehäuseluftauslass (22) aufweist, der durch den nachgeschalteten Wandabschnitt verläuft.

15. Patronenanordnung (10) nach einem der vorhergehenden Ansprüche, ferner aufweisend eine Dichtung (36), die sich über das zuströmseitige Ende der Patrone (14) erstreckt.

16. Patronenanordnung (10) nach einem der vorhergehenden Ansprüche, wobei die Patrone (14) weiter eine Nikotinquelle (78), die innerhalb der ersten Kammer (44) angeordnet ist, und eine Säurequelle (80), die innerhalb der zweiten Kammer (46) angeordnet ist, aufweist.

17. Patronenanordnung (10) nach einem der vorhergehenden Ansprüche, wobei die Patrone (14) eine dritte Kammer (48) zur Aufnahme eines Heizelements einer Aerosolerzeugungsvorrichtung aufweist, wobei die dritte Kammer (48) eine Kammeröffnung am zuströmseitigen Ende der Patrone (14) aufweist, und wobei die dritte Kammer zwischen der ersten Kammer (44) und der zweiten Kammer (46) angeordnet ist.

18. Patronenanordnung (10) nach einem der Ansprüche 1 bis 16, wobei die Patrone (14) einen Suszeptor aufweist, der zwischen der ersten Kammer (44) und der zweiten Kammer (46) angeordnet ist.

19. Aerosolerzeugungssystem, aufweisend:
eine Patronenanordnung (10) nach einem der Ansprüche 1 bis 16; und
eine Aerosolerzeugungsvorrichtung, die einen Vorrichtungshohlraum, der ausgelegt ist, ein zuströmseitiges Ende der Patronenanordnung (10) aufzunehmen, und eine Heizvorrichtung zum Erwärmen der ersten Kammer (44) und der zweiten Kammer (46) der Patrone (14) der Patronenanordnung (10) aufweist.

20. Aerosolerzeugungssystem nach Anspruch 19, wobei die Heizvorrichtung ein innerhalb des Vorrichtungshohlraums angeordnetes Heizelement aufweist, wobei die Patrone (14) eine dritte Kammer (48) zur Aufnahme des Heizelements aufweist, wobei die dritte Kammer (48) eine Kammeröffnung am zuströmseitigen Ende der Patrone (14) aufweist, und wobei die dritte Kammer (48) zwischen der ersten Kammer (44) und der zweiten Kammer (46) angeordnet ist.

21. Aerosolerzeugungssystem nach Anspruch 19, wobei die Heizvorrichtung eine induktive Heizvorrichtung aufweist, die zumindest einen Abschnitt des Vorrichtungshohlraums umgibt, und wobei die Patrone (14) einen Suszeptor aufweist, der zwischen der ersten Kammer (44) und der zweiten Kammer (46) angeordnet ist.

## Revendications

1. Ensemble de cartouche (10) destiné à être utilisé dans un système de génération d'aérosol, l'ensemble de cartouche (10) comprenant :
un logement d'embout buccal (12) ayant une extrémité amont et une extrémité aval, le logement d'embout buccal (12) définissant une cavité d'embout buccal (20) s'étendant entre l'extrémité amont du logement d'embout buccal (12) et l'extrémité aval du logement d'embout buccal (12) ;
un élément de blocage (26) fixé à l'intérieur de la cavité d'embout buccal (20) et comprenant une extrémité amont et une extrémité aval ; et
une cartouche (14) positionnée au niveau de l'extrémité amont du logement d'embout buccal (12), la cartouche (14) comprenant :
une extrémité amont et une extrémité aval ;
un premier compartiment (44) ayant une première entrée d'air (62) au niveau de l'extrémité amont de la cartouche (14) et une première sortie d'air (92) au niveau de l'extrémité aval de la cartouche (14) ; et
un deuxième compartiment (46) ayant une deuxième entrée d'air (64) au niveau de l'extrémité amont de la cartouche (14) et une deuxième sortie d'air (94) au niveau de l'extrémité aval de la cartouche (14) ;
dans lequel l'extrémité aval de la cartouche (14) bute contre l'extrémité amont de l'élément de blocage (26) ;
dans lequel la cartouche (14) peut tourner par rapport à l'élément bloquant (26) d'une première position à une deuxième position ; et
dans lequel l'élément de blocage (26) est formé de sorte que l'extrémité amont de l'élément de blocage (26) obstrue la première sortie d'air (92) et la deuxième sortie d'air (94) lorsque la cartouche (14) est dans la première position, et la première sortie d'air (92) et la deuxième sortie d'air (94) sont toutes deux en communication fluidique avec la cavité d'embout buccal (20) lorsque la cartouche (14) est dans la deuxième position.

2. Ensemble de cartouche (10) selon la revendication 1, dans lequel l'un parmi l'élément de blocage (26) et la cartouche (14) comprend une ouverture (96) et dans lequel l'autre parmi l'élément de blocage (26) et la cartouche (14) comprend un arbre (84), dans lequel au moins une partie de l'arbre (84) est positionnée à l'intérieur de l'ouverture (96).

3. Ensemble de cartouche (10) selon la revendication 2, dans lequel l'ouverture (96) s'étend à travers l'élément de blocage (26) entre l'extrémité amont de l'élément de blocage (26) et l'extrémité aval de l'élément de blocage (26), et dans lequel l'arbre (84) s'étend depuis l'extrémité aval de la cartouche (14).

4. Ensemble de cartouche (10) selon la revendication 2 ou 3, dans lequel l'arbre (84) comprend une partie de tourillon (86) positionnée à l'intérieur de l'ouverture (96) et une partie de retenue (88) à une extrémité de la partie de tourillon (86), dans lequel la partie de retenue (88) est positionnée à l'extérieur de l'ouverture (96) et a un diamètre qui est plus grand qu'un diamètre de l'ouverture (96).

5. Ensemble de cartouche (10) selon l'une quelconque des revendications précédentes, dans lequel la cartouche (14) comprend une broche de guidage (90) s'étendant à partir de l'extrémité aval de la cartouche (14), dans lequel la broche de guidage (90) bute contre une première partie de l'élément de blocage (26) lorsque la cartouche (14) est dans la première position, et dans lequel la broche de guidage (90) bute contre une deuxième partie de l'élément de blocage (26) lorsque la cartouche (14) est dans la deuxième position.

6. Ensemble de cartouche (10) selon l'une quelconque des revendications précédentes, dans lequel la cartouche (14) comprend :
un logement de cartouche (32) positionné au niveau de l'extrémité amont du logement d'embout buccal (12) et pouvant tourner par rapport à l'élément de blocage (26) de la première position à la deuxième position ; et
un corps de cartouche (34) fixé à l'intérieur du logement de cartouche (32), le corps de cartouche (34) définissant le premier compartiment (44) et le deuxième compartiment (46).

7. Ensemble de cartouche (10) selon l'une quelconque des revendications précédentes, dans lequel l'élément de blocage (26) est espacé de l'extrémité aval du logement d'embout buccal (12), et dans lequel une partie de la cavité d'embout buccal (20) entre l'extrémité aval de l'élément de blocage (26) et l'extrémité aval du logement d'embout buccal (12) forme une chambre de mélange.

8. Ensemble de cartouche (10) selon la revendication 7, dans lequel le logement d'embout buccal (12) comprend une entrée d'air de ventilation positionnée entre l'élément de blocage (26) et l'extrémité aval du logement d'embout buccal (12), l'entrée d'air de ventilation s'étendant à travers le logement d'embout buccal (12) et fournissant une communication fluidique entre l'extérieur du logement d'embout buccal (12) et la chambre de mélange.

9. Ensemble de cartouche (10) selon l'une quelconque des revendications précédentes, dans lequel l'élément de blocage (26) comprend une première ouverture d'écoulement d'air (98) configurée pour recouvrir la première sortie d'air (92) lorsque la cartouche (14) est dans la deuxième position et une deuxième ouverture d'écoulement d'air (100) configurée pour recouvrir la deuxième sortie d'air (94) lorsque la cartouche (14) est dans la deuxième position.

10. Ensemble de cartouche (10) selon la revendication 9, dans lequel l'élément de blocage (26) a une forme circulaire et dans lequel la première ouverture d'écoulement d'air (98) et la deuxième ouverture d'écoulement d'air (100) ont chacune une forme arquée.

11. Ensemble de cartouche (10) selon la revendication 10, dans lequel la première ouverture d'écoulement d'air (98) et la deuxième ouverture d'écoulement d'air (100) sont diamétralement opposées.

12. Ensemble de cartouche (10) selon la revendication 10 ou 11, dans lequel la première ouverture d'écoulement d'air (98) et la deuxième ouverture d'écoulement d'air (100) s'étendent chacune le long d'une longueur d'arc de quart de cercle.

13. Ensemble de cartouche (10) selon l'une quelconque des revendications 9 à 12, dans lequel l'élément de blocage (26) comprend une première partie de blocage (102) positionnée entre une première extrémité de la première ouverture d'écoulement d'air (98) et une deuxième extrémité de la deuxième ouverture d'écoulement d'air (100), dans lequel l'élément de blocage (26) comprend une deuxième partie de blocage (104) positionnée entre une deuxième extrémité de la première ouverture d'écoulement d'air (98) et une première extrémité de la deuxième ouverture d'écoulement d'air (100), dans lequel la première partie de blocage (102) est configurée pour obstruer la première sortie d'air (92) lorsque la cartouche (14) est dans la première position, et dans lequel la deuxième partie de blocage (104) est configurée pour obstruer la deuxième sortie d'air (94) lorsque la cartouche (14) est dans la première position.

14. Ensemble de cartouche (10) selon l'une quelconque des revendications précédentes, dans lequel le logement d'embout buccal (12) comprend une partie de paroi en aval s'étendant à travers l'extrémité aval de la cavité d'embout buccal (20), le logement d'embout buccal (12) comprenant une sortie d'air de logement d'embout buccal (22) s'étendant à travers la partie de paroi en aval.

15. Ensemble de cartouche (10) selon l'une quelconque des revendications précédentes, comprenant en outre un joint (36) s'étendant à travers l'extrémité amont de la cartouche (14).

16. Ensemble de cartouche (10) selon l'une quelconque des revendications précédentes, dans lequel la cartouche (14) comprend en outre une source de nicotine (78) positionnée à l'intérieur du premier compartiment (44) et une source d'acide (80) positionnée à l'intérieur du deuxième compartiment (46).

17. Ensemble de cartouche (10) selon l'une quelconque des revendications précédentes, dans lequel la cartouche (14) comprend un troisième compartiment (48) pour recevoir un élément de chauffage d'un dispositif de génération d'aérosol, dans lequel le troisième compartiment (48) comprend une ouverture de compartiment au niveau de l'extrémité amont de la cartouche (14), et dans lequel le troisième compartiment est positionné entre le premier compartiment (44) et le deuxième compartiment (46).

18. Ensemble de cartouche (10) selon l'une quelconque des revendications 1 à 16, dans lequel la cartouche (14) comprend un suscepteur positionné entre le premier compartiment (44) et le deuxième compartiment (46).

19. Système de génération d'aérosol comprenant :
un ensemble de cartouche (10) selon l'une quelconque des revendications 1 à 16 ; et
un dispositif de génération d'aérosol comprenant une cavité de dispositif configurée pour recevoir une extrémité amont de l'ensemble de cartouche (10) et un dispositif de chauffage pour le chauffage du premier compartiment (44) et du deuxième compartiment (46) de la cartouche (14) de l'ensemble de cartouche (10).

20. Système de génération d'aérosol selon la revendication 19, dans lequel le dispositif de chauffage comprend un élément de chauffage positionné à l'intérieur de la cavité du dispositif, dans lequel la cartouche (14) comprend un troisième compartiment (48) pour recevoir l'élément de chauffage, dans lequel le troisième compartiment (48) comprend une ouverture de compartiment au niveau de l'extrémité amont de la cartouche (14), et dans lequel le troisième compartiment (48) est positionné entre le premier compartiment (44) et le deuxième compartiment (46).

21. Système de génération d'aérosol selon la revendication 19, dans lequel le dispositif de chauffage comprend un dispositif de chauffage à induction entourant au moins une partie de la cavité de dispositif, et dans lequel la cartouche (14) comprend un suscepteur positionné entre le premier compartiment (44) et le deuxième compartiment (46).
